# EUROPÄISCHE PATENTSCHRIFT

(11) **EP 3 831 415 B1**
(45) Veröffentlichungstag und Bekanntmachung des Hinweises auf die Patenterteilung: **01.05.2024**
(21) Anmeldenummer: 20207661.8
(22) Anmeldetag: 13.11.2020
(51) Int. Cl.: A61L 2/10, B66B 11/02

(54) **VORRICHTUNG UND VERFAHREN ZUR DESINFEKTION EINES RAUMES**
METHOD AND DEVICE FOR DISINFECTING AN AREA
DISPOSITIF ET PROCÉDÉ DE DÉSINFECTION D'UN ESPACE

(30) Priorität: 13.11.2019 DE 102019130620
(43) Veröffentlichungstag der Anmeldung: 09.06.2021
(73) Patentinhaber: Uventions GmbH, 20537 Hamburg (DE)
(72) Erfinder: EHLERS, Daniel, 22529 Hamburg (DE); GROßKLAUS, Axel Gordon, 20359 Hamburg (DE)
(74) Vertreter: Gulde & Partner

(56) Entgegenhaltungen:
- CN-A- 105 800 426
- CN-U- 203 682 802
- CN-U- 207 658 902
- CN-U- 207 792 412

## Beschreibung

Die vorliegende Erfindung betrifft eine Vorrichtung und ein Verfahren zur Desinfektion eines Raums, insbesondere einer Oberfläche innerhalb des Fahrkorbs einer Aufzugsanlage, mit mindestens einer UV-Lichtquelle, die zur Emission von UV-Licht zur Desinfektion des Raums ausgebildet ist.

Aufzüge sind heute in einer Vielzahl von Gebäuden zur Beförderung von Personen, Lasten und Gütern installiert und im Betrieb. In den meisten Fällen werden die Aufzüge von vielen verschiedenen Personen genutzt. Bei der Nutzung des Aufzugs ist es in den meisten Fällen erforderlich die Oberflächen des Tableaus des Aufzugs zu berühren, um die gewünschte Zielhaltestelle einzugeben. Außerdem berühren viele Nutzer die Oberflächen des Handlaufs oder stützen sich an den Fahrkorbwänden ab, um sich sicheren Halt während der Fahrt zu verschaffen. Des Weiteren werden in Aufzugsfahrkörben zum Beispiel auch benutzte Taschentücher, ausgespuckte Kaugummis oder andere kontaminierte Gegenstände liegen gelassen. In extremen Fällen sind beispielsweise auch Körperflüssigkeiten wie Urin menschlicher und tierischer Herkunft in den Fahrkörben von Aufzugsanlagen vorzufinden. Diese und weitere Kontaminierungen mit Bakterien, Viren, Pilzen, Hefen und/oder Keimen setzen die Nutzer von Aufzügen bei einer Berührung von Oberflächen einem erhöhten infektions-Risiko aus.

In der Regel werden Aufzüge im Rahmen der normalen Gebäudereinigung gereinigt. Häufig wird dabei nur der Fahrkorbboden gereinigt. Aber auch wenn die Bedienelemente, Handläufe und/oder Fahrkorbwände gereinigt werden, werden diese häufig ohne spezielle Reinigungsmittel zur Reduzierung von Krankheitserregern gereinigt. Chemische Reinigungsmittel zur Desinfektion von Oberflächen hinterlassen häufig Rückstände und auch Gerüche.

In medizinischen Einrichtungen wird seit geraumer Zeit UV-Licht zur Desinfektion und Sterilisierung z. B. von medizinischen Instrumenten erfolgreich eingesetzt. Der Vorteil des Einsatzes von UV-Licht bei der Sterilisierung ist, dass dieses in der Regel weit über 90 % der Krankheitserreger vernichtet und keine zusätzlichen chemischen oder biologischen Zusatzstoffe zur Anwendung kommen. Sogar multiresistente Keime, wie sie in Krankenhäusern vorkommen, können zum Teil so vernichtet werden. Beispielsweise kommt UV-Licht derzeit bei der Desinfektion von Krankenzimmern in Krankenhäusern zur Anwendung.

Da erhöhte Strahlendosen der UV-C-Strahlen beim Menschen Hautrötungen (Erytheme) und schmerzhafte Augenentzündungen (Konjunktivitis) verursachen, dürfen Personen diesen Strahlen nicht ausgesetzt sein beziehungsweise werden.

Es ist Aufgabe der vorliegenden Erfindung, sich die Idee der UV-Licht Desinfektion im Bereich der Aufzugsanlagen zu Nutze zu machen und eine Vorrichtung beziehungsweise ein Verfahren bereitzustellen, die die Desinfektion von Oberflächen in geschlossenen oder offenen Räumen, vorzugsweise in Aufzügen, mittels UV-C-Licht- sofern sich keine Personen im Strahlungsbereich aufhalten - erlauben.

Das Gebrauchsmuster CN 203 682 802 U offenbart eine Aufzugsumgebungskonditionierungs- und Sicherheitsvorwarnvorrichtung und bezieht sich auf das technische Gebiet der Aufzugsumgebungsüberwachung. Die Vorrichtung ist mit einem Ein-Chip-Mikrocomputer, einem Sensordetektionsteil und einem Aktuatorteil versehen, wobei der Sensordetektionsteil und der Aktuatorteil jeweils mit dem Ein-Chip-Mikrocomputer verbunden sind; der Sensordetektionsteil umfasst einen Temperatur- und Feuchtigkeitssensor, ein Rauchsensormodul, ein Modul für die Infrarotinduktion des menschlichen Körpers, einen Lichtintensitätssensor, einen Beschleunigungssensor und ein Schallsensormodul; der Aktuatorteil umfasst ein Wechselstromgebläse, eine Desinfektionslampe für ultraviolette Strahlung, ein Sprachaufforderungsmodul und einen Wärmer; der Ein-Chip-Mikrocomputer ist auch mit einer Taste verbunden, die zur Betriebssteuerung verwendet wird. Die Aufzugsumgebungskonditionierungs- und Sicherheitsvorwarnvorrichtung hat den Vorteil, dass die Aufzugsfahrt angenehmer ist und der Aufzugsbetrieb sicherer, zuverlässiger und energiesparender ist.

Das Gebrauchsmuster CN 207 658 902 U offenbart eine Art von multifunktionalen Aufzug Pendellampe, einschließlich Lampenschirm, obere Platte und Leiterplatte, Lampenschirm ist durch Bolzen mit der oberen Platte zu verbinden, und die Leiterplatte ist in Lampenschirm montiert, und ultraviolette Desinfektion Lampe und LED-Beleuchtung Lampe sind in Leiterplatte unteren Oberfläche ausgestattet. Die multifunktionale Aufzug Hängelampe des Gebrauchsmusters hat die Funktion der Beleuchtung und ultraviolette Lampe Antivirus, menschlicher Körpersensor ist auf Hängelampe vorgesehen, durch menschlichen Körpersensor erkennen Aufzug Kabine in, ob jemand, Mikrocontroller steuert die offenen und geschlossenen Zustand der ultravioletten Desinfektionslampe nach der Erkennung Übergang des menschlichen Körpers Sensor; Das Gebrauchsmuster hat auch Rauchen Warnfunktion, Wenn der Raucherkennungssensor feststellt, dass jemand in der Aufzugskabine raucht, meldet der Mikrocontroller über den Lautsprecher "Rauchen verboten" und warnt den Raucher.

CN 105 800 426 A stellt einen Aufzug zur Verfügung, umfassend einen Drucksensor, einen Sauerstoffkonzentrationssensor, einen Rauchsensor, eine Eingabevorrichtung, eine Identifikationsvorrichtung, einen Lautsprecher, eine Kamera, eine Luftwechselvorrichtung, eine ultraviolette Keimtötungslampe und einen Mikroprozessor. Gemäß dem neuartigen Aufzug wird der Mikroprozessor dazu verwendet, die Signale aller Sensoren zu integrieren und zu analysieren und gleichzeitig entsprechende Anweisungen zu erteilen, wodurch eine intelligente Steuerung erreicht werden kann; die Eingabevorrichtung kann für einen Notruf verwendet werden und kann auch einfach vom Arbeitspersonal zur gleichen Zeit bedient werden, um die Anweisungen zu ändern; wenn der Drucksensor feststellt, dass der Druckwert im Aufzug der Anfangswert ist, d.h. keine Person den Aufzug benutzt, wird die ultraviolette keimtötende Lampe so gesteuert, dass sie in diesem Moment arbeitet, um den Aufzug zu desinfizieren; wenn ein Schwerkraftsensor feststellt, dass die Schwerkraft des Aufzugs abnormal ist, wird eine Verriegelungsvorrichtung gesteuert, um eine Sicherheitsbehandlung am Aufzug durchzuführen; und der neuartige Aufzug ist einfach in der Struktur, in der Lage, Intelligenz zu erreichen und das Kundenerfahrungsgefühl zu verbessern, sicherer und sehr hoch in der Praktikabilität.

CN 207 792 412 U offenbart eine Art von sprachinteraktiven intelligenten Aufzügen, einschließlich Aufzug, terrassenförmiges Gehäuse ist im Aufzug vorgesehen, es ist mit Umlenkrolle auf der seitlichen Oberfläche der linken Seitenplatte des Leitergehäuses installiert, es ist mit Anti-Fall-Mechanismus auf dem Leitergehäuse vorgesehen, Controller ist auf der seitlichen Oberfläche der oberen Platte des Leitergehäuses installiert, es ist mit Temperatursensor auf der medialen Oberfläche der oberen Platte des Leitergehäuses installiert, Es ist mit LED-Anzeige und Infrarot-Sensor auf der mittleren Oberfläche der Rückseite der Leiter Fall installiert ist, ist es mit Spracherkennung Ausrüstung auf der mittleren Oberfläche der rechten Platte der Leiter Fall installiert ist, ist es mit augenblicklichen Sprachleistung auf der linken Seitenplatte der Leiter Fall installiert ist, ist es mit elektrischen Heizlampe auf der linken Seitenplatte der Leiter Fall und der mittleren Oberfläche der Rückseite installiert.

### Offenbarung der Erfindung

Ein Aspekt der Erfindung betrifft eine Vorrichtung zur Desinfektion mindestens einer Oberfläche innerhalb des Fahrkorbs einer Aufzugsanlage. Dabei umfasst die Vorrichtung: mindestens eine UV-Lichtquelle, die zur Emission von UV-Licht zur Desinfektion der Oberfläche innerhalb des Fahrkorbs ausgebildet ist;
mindestens einen Anwesenheitsdetektor, der dazu ausgebildet ist, die Anwesenheit mindestens eines Lebewesens innerhalb des Fahrkorbs zu erkennen und im Falle der Erkennung der Anwesenheit mindestens eines Lebewesens innerhalb des Fahrkorbs ein die Anwesenheit des Lebewesens indizierendes Sensorsignal zu erzeugen;
mindestens einen Beschleunigungssensor, welcher dazu ausgebildet ist, zu erkennen, wenn sich die Vorrichtung horizontal und/oder vertikal bewegt und ein diese Bewegung indizierendes Sensorsignal auszugeben; und
mindestens eine mit dem mindestens einen Anwesenheitsdetektor und dem mindestens einen Beschleunigungssensor und der mindestens einen UV-Lichtquelle verbundene Steuereinheit. Erfindungsgemäß ist die mindestens eine Steuereinheit dazu ausgebildet, den Betrieb der UV-Lichtquelle zu steuern und sowohl auf den Empfang des die Anwesenheit des Lebewesens indizierenden Sensorsignals, wie auch auf den Empfang des die Bewegung indizierenden Sensorsignals hin eine Initiation eines Betriebes der UV-Lichtquelle zu unterbinden und/oder einen laufenden Betrieb der UV-Lichtquelle zu unterbrechen.

Ein besonderer Vorteil dieser Ausführungsform besteht in der Kombination mehrerer Sensoren.

Bevorzugt wird ferner eine Aufzugsanlage mit einer erfindungsgemäßen Vorrichtung zur Verfügung gestellt.

Ein weiterer Aspekt der Erfindung betrifft ein Verfahren zur Desinfektion mindestens einer Oberfläche innerhalb des Fahrkorbs einer Aufzugsanlage, mit mindestens einer UV-Lichtquelle sowie mindestens einem Anwesenheitsdetektor und mindestens einem Beschleunigungssensor. Dabei umfasst das erfindungsgemäße Verfahren die folgenden Schritte:
- Emittieren (S1) von UV-Licht auf die zu desinfizierende Oberfläche des Fahrkorbs mittels der mindestens einen UV-Lichtquelle;
- Erkennen (S2) der Anwesenheit mindestens eines Lebewesens innerhalb des Fahrkorbs mittels des mindestens einen Anwesenheitsdetektors;
- Erkennen (S3) der horizontalen und/oder vertikalen Bewegung der Aufzugsanlage mittels des mindestens einen Beschleunigungssensors,
- Unterbrechen (S4) der Emission des UV-Lichtes, sowohl auf den Empfang des die Anwesenheit des Lebewesens indizierenden Sensorsignals, wie auch wenn mittels des mindestens einen Beschleunigungssensors eine horizontale und/oder vertikale Bewegung der Aufzugsanlage erkannt wird.

Weitere vorteilhafte Weiterbildungen der Erfindung sind in den Unteransprüchen angegeben und in der Beschreibung beschrieben.

Als UV-C-Licht ist im Sinne dieser Erfindung Licht mit einer Wellenlänge von 200-280nm zu verstehen. Als Lichtquelle kommen LEDs aber auch UV-C-Nieder- und Mitteldruckstrahler in der oben genannten Bandbreite zur Anwendung.

Um zu vermeiden, dass Personen und/oder Tiere der UV-C-Strahlung ausgesetzt werden kommen mehrere und verschiedene Sensoren zum Einsatz, deren Signale von einer Steuerbeziehungsweise einer Kontrolleinheit, insbesondere einem Controller, ausgewertet werden.

Die Erfassung einer Situation, in welcher sich bewegende Personen (eine oder mehrere) oder Tiere im Strahlungsbereich aufhalten und wie sie diesen Bereich zeitlich/räumlich nutzen und ggf. mit Pathogenen kontaminieren und einen anschließenden Desinfektionsvorgang notwendig machen, kann aktiv mit elektromagnetischen Wellen, insbesondere unter Verwendung von Hochfrequenz-, HF-, Wellen und/oder Mikrowellen/Dopplerradar), mit Ultraschall (Ultraschall-Bewegungsmelder) oder über einen Infrarot-Sensor aktiv (Infrarot-Laser bei dem Laufzeiten nach dem Time-of-Flight- oder Lidar-Prinzip zu einem 3D-Bild der Umgebung zusammengesetzt werden, nachfolgend auch "3D Kamera") oder passiv (PIR oder Infrarot-Sensorik-Array, "Infrarot-Kamera") anhand der Infrarotstrahlung der Umgebung erfolgen. Auch eine Detektion mittels Foto Zellen-Bewegungsmelder kann zum Einsatz kommen. Die Aufzählung der möglichen Bewegungssensoren ist nicht als vollständig und abschließend zu betrachten.

Zusätzlich zum Bewegungssensor ist ein Sensor zur Detektion von anwesenden Personen/Tieren installiert, der ermittelt, ob sich im Strahlungsbereich Personen oder Tiere aufhalten, die sich über einen längeren Zeitraum nicht bewegt haben (zum Beispiel schlafende Personen oder Personen, die das Bewusstsein verloren haben) und somit vom Bewegungssensor gegebenenfalls nicht erkannt werden. Dafür können zum Beispiel Laserscanner, Stereo-Kameras, 3D-Kameras, Time-of-Flight-3D-Kameras oder aktive Triangulationssysteme zum Einsatz kommen. Diese Aufzählung ist nicht als vollständig und abschließend zu betrachten.

Als dritter Sensor ermittelt ein Beschleunigungssensor, beispielsweise ein 3-Achsen-Accelerometer, ob sich der zu bestrahlende Raum horizontal (zum Beispiel ein Container oder Laderaum eines Fahrzeuges) oder vertikal (zum Beispiel ein Aufzug) bewegt. Ob und wie die Signale des Accelerometers vom Controller interpretiert werden, wird im Rahmen der Installation/Kalibrierung des Gerätes festgelegt.

Alle Sensoren sind mit einem Controller verbunden, der die Signale der Sensoren auswertet und ermittelt, ob sich eine Person / ein Tier im Strahlungsbereich aufhält. Die Verbindung zwischen den Sensoren und dem Controller kann sowohl kabelgebunden wie auch kabellos realisiert werden. Bei der kabellosen Übertragung kann es sich Bluetooth, Bluetooth LE, WLAN, ZIGBEE, LORA, Z-Wave, 6LowPAN, Thread, Sigfox, NFC, GSM, UMTS, LTE oder 5G handeln. Diese Aufzählung ist nicht als vollständig und abschließend zu betrachten.

Nur wenn der Controller feststellt, dass sich keine bewegende Person/kein bewegendes Tier und/oder keine Person/kein Tier welche/welches sich über einen längeren Zeitraum nicht bewegt hat, im Bestrahlungsraum anwesend ist, wird der Bestrahlung Vorgang über die UV-C-Leuchtmittel initiiert. Für den Einsatz im Aufzug wird durch das Accelerometer ergänzend detektiert, ob sich der Fahrkorb in Fahrt befindet, so dass der Bestrahlungsvorgang ebenfalls unterbrochen wird, da selbst bei nicht anwesenden Personen im Falle einer Fahrt davon ausgegangen werden kann, dass in Kürze eine Person den Fahrkorb betritt.

Da die Desinfektionswirkung sowohl von der Strahlendosis, der Dauer der Bestrahlung sowie der Entfernung der emittierenden Strahlungsquelle und der zu desinfizierenden Oberfläche abhängig ist, kann die Dauer der Bestrahlung für eine vollständige Desinfektion im Rahmen der Installation/Kalibrierung des Gerätes festgelegt werden.

Die Bestrahlungsvorgänge (Datum und Uhrzeit des Beginns, Datum und Uhrzeit der Beendigung) werden in einem Speicher protokolliert. Sofern ein Bestrahlungsvorgang durch die Detektion eines Sensors unterbrochen wird, wird auch der Grund der Unterbrechung protokolliert. Die Protokolle können über eine kabelgebundene oder kabellose Kommunikationsschnittstelle auf ein anderes Gerät (Handy, Tablet, PC) übertragen werden. Auch Fehlermeldungen des/der Controller(s) können so übertragen werden. Bei der kabellosen Übertragung kann es sich Bluetooth, Bluetooth LE, WLAN, ZIGBEE, LORA, Z-Wave, 6LowPAN, Thread, Sigfox, NFC, GSM, UMTS, LTE oder 5G handeln. Diese Aufzählung ist nicht als vollständig und abschließend zu betrachten.

Sämtliche Sensoren sowie auch der Controller können aber müssen nicht aus Sicherheitsgründen redundant ausgeführt werden. In diesem Fall gleichen die Controller die empfangenen Daten der Sensoren ab. Nur wenn beide Controller feststellen, dass sich keine bewegende Person/kein bewegendes Tier und/oder keine Person/kein Tier welche/welches sich über einen längeren Zeitraum nicht bewegt hat im Bestrahlungsraum anwesend ist, wird der Bestrahlung Vorgang über die UV-C-Leuchtmittel initiiert. Zusätzlich überwachen sich die beiden Controller gegenseitig auf mögliche Systemfehler. Stellt ein Controller beim zweiten Controller einen Fehler fest, wird kein weiterer Bestrahlungsvorgang mehr initiiert, bis der Fehler behoben wurde.

Sämtliche Sensoren, der/die Controller sowie die Lichtquelle können gemeinsam in einem Gehäuse verbaut werden. Alternativ können sämtliche Sensoren, der/die Controller und auch die Lichtquelle separat und räumlich voneinander getrennt installiert werden. Die Verbindung der zuvor genannten Komponenten kann sowohl Kabel gebunden wie auch kabellos realisiert werden.

Die UV-C-Lichtquelle kann an der Decke des Raumes oder an der Wand installiert werden.

Generelle Vorteile der Desinfektion mittels UV-Strahlung gegenüber chemischen Desinfektionsverfahren ist, dass sie vollständig rückstandsfrei, geruchsfrei und umweltfreundlicher ist.

Die Verwendung von LEDs als Lichtquelle hat gegenüber den UV-C-Nieder- und Mitteldruckstrahlern den Vorteil, dass LEDs keine Einschaltverzögerung haben, eine längere Lebensdauer haben, weniger Energie verbrauchen, keine Schadstoffe oder giftigen Substanzen enthalten und erheblich robuster und stoßfester sind.

Das hier vorgestellte Verfahren hat gegenüber der manuellen chemischen Desinfektion den weiteren Vorteil, dass es automatisch und ohne menschlichen Eingriff erfolgt und somit gegenüber einer Desinfektion durch Reinigungspersonal als zuverlässiger zu betrachten ist und eine Protokollierung automatisch erfolgt. Beim (Teil-)Entfall von manuellen Desinfektionen werden so entsprechend Personal- und Materialkosten reduziert.

Die Erfindung soll nachstehend anhand von einem zumindest teilweise in der Figur dargestellten Ausführungsbeispiel näher erläutert werden.

Es zeigt:

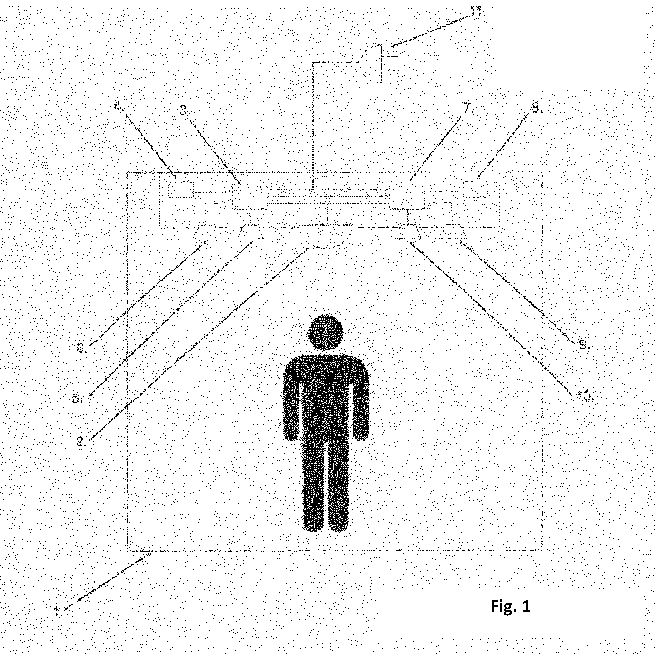

Figur 1 Eine Prinzipdarstellung der Elemente zur Desinfektion eines Aufzugfahrkorbes.

Wie aus Fig. 1 zu ersehen ist, ist in einem Aufzugsfahrkorb 1 eine UV-Lichtquelle 2, die zur Emission von UV-Licht zur Desinfektion der Oberfläche innerhalb des Raums ausgebildet ist, angeordnet. Die UV-Lichtquelle 2 ist im vorliegenden Ausführungsbeispiel als UV-C LED Leuchtmittel ausgebildet.

Um zu vermeiden, dass Personen und/oder Tiere der UV-C-Strahlung ausgesetzt werden, kommen mehrere und verschiedene Sensoren zum Einsatz, deren Signale von einer Steuerbeziehungsweise einer Kontrolleinheit, im vorliegenden Ausführungsbeispiel von zwei Controllern 3 und 7, ausgewertet werden.

Ein Anwesenheitsdetektor, der dazu ausgebildet ist, die Anwesenheit mindestens eines Lebewesens innerhalb des Raums zu erkennen und im Falle der Erkennung der Anwesenheit mindestens eines Lebewesens innerhalb des Raums ein die Anwesenheit des Lebewesens indizierendes Sensorsignal zu erzeugen, weist im vorliegenden Ausführungsbeispiel die Bewegungssensor 5 und10 auf, welche zur Detektion einer Bewegung mindestens eines innerhalb des Raums anwesenden Lebewesens ausgebildet sind.

Weiterhin sind Nicht-Bewegungssensoren 6 und 9 angeordnet, welche dazu ausgebildet sind, die Anwesenheit mindestens eines sich nicht bewegenden Lebewesens innerhalb des Raums zu erkennen.

Zusätzlich sind Beschleunigungssensoren 4 und 8 angeordnet, welche dazu ausgebildet sind, zu erkennen, wenn sich die Vorrichtung horizontal und/oder vertikal bewegt und ein diese Bewegung indizierendes Sensorsignal auszugeben.

Eine Steuereinheit mit den Controllern 3 und 7 ist dazu ausgebildet, den Betrieb der UV-Lichtquelle 2 zu steuern und auf den Empfang des die Anwesenheit des Lebewesens indizierenden Sensorsignals und/oder des die Bewegung indizierenden Sensorsignals hin eine Initiation eines Betriebes der UV-Lichtquelle 2 zu unterbinden und/oder einen laufenden Betrieb der UV-Lichtquelle 2 zu unterbrechen. Über einen Netzstromstecker 11 erfolgt die Energieversorgung.

Obwohl die Erfindung im Detail durch bevorzugte Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Vorrichtung zur Desinfektion mindestens einer Oberfläche innerhalb des Fahrkorbs (1) einer Aufzugsanlage, wobei die Vorrichtung umfasst:
mindestens eine UV-Lichtquelle (2), die zur Emission von UV-Licht zur Desinfektion der Oberfläche innerhalb des Fahrkorbs (1) ausgebildet ist;
mindestens einen Anwesenheitsdetektor, der dazu ausgebildet ist, die Anwesenheit mindestens eines Lebewesens innerhalb des Fahrkorbs (1) zu erkennen und im Falle der Erkennung der Anwesenheit mindestens eines Lebewesens innerhalb des Fahrkorbs (1) ein die Anwesenheit des Lebewesens indizierendes Sensorsignal zu erzeugen;
mindestens einen Beschleunigungssensor (4, 8), welcher dazu ausgebildet ist, zu erkennen, wenn sich die Vorrichtung horizontal und/oder vertikal bewegt und ein diese Bewegung indizierendes Sensorsignal auszugeben; und
mindestens eine mit dem mindestens einen Anwesenheitsdetektor und dem mindestens einen Beschleunigungssensor (4, 8) und der mindestens einen UV-Lichtquelle (2) verbundene Steuereinheit (3, 7),
**dadurch gekennzeichnet, dass**
die obengenannte Vorrichtung dazu ausgebildet ist, den Betrieb der UV-Lichtquelle (2) zu steuern und sowohl auf den Empfang des die Anwesenheit des Lebewesens indizierenden Sensorsignals, wie auch auf den Empfang des die Bewegung indizierenden Sensorsignals hin eine Initiation eines Betriebes der UV-Lichtquelle (2) zu unterbinden und/oder einen laufenden Betrieb der UV-Lichtquelle (2) zu unterbrechen.

2. Vorrichtung nach Anspruch 1, wobei der mindestens eine Anwesenheitsdetektor mindestens einen Bewegungssensor (5, 10) umfasst, welcher zur Detektion einer Bewegung mindestens eines innerhalb des Fahrkorbs (1) anwesenden Lebewesens ausgebildet ist.

3. Vorrichtung nach Anspruch 2, wobei der mindestens eine Bewegungssensor (5, 10) zur Emission und/oder zum Empfang von elektromagnetischer Strahlung, insbesondere von Strahlung im Hochfrequenzbereich, im Mikrowellenbereich und/oder im Ultraschallbereich ausgebildet ist.

4. Vorrichtung nach Anspruch 2 oder 3, wobei der mindestens eine Bewegungssensor (5, 10) ein Doppler-Radar, einen Ultraschall-Bewegungsmelder, einen Pyroelektrischen Sensor und/oder einen Fotozellen-Bewegungsmelder umfasst.

5. Vorrichtung nach einem der vorhergehenden Ansprüche, wobei der mindestens eine Anwesenheitsdetektor mindestens einen Nicht-Bewegungssensor (6, 9) umfasst, welcher dazu ausgebildet ist, die Anwesenheit mindestens eines sich nicht bewegenden Lebewesens innerhalb des Fahrkorbs (1) zu erkennen.

6. Vorrichtung nach Anspruch 5, wobei der mindestens eine Nicht-Bewegungssensor (6, 9) einen Laserscanner, eine Stereo-Kamera, eine 3D-Kamera, eine Time-of-Flight-3D-Kamera und/oder ein aktives Triangulationssystem umfasst.

7. Vorrichtung nach Anspruch 1, wobei der mindestens eine Beschleunigungssensor (4, 8) als Accelerometer, insbesondere als 3-Achsen-Accelerometer, ausgeführt ist.

8. Vorrichtung nach Anspruch 1 oder 7, wobei die Steuereinheit (3, 7) ferner dazu ausgebildet ist, auf den Empfang des die Bewegung der Aufzugsanlage indizierenden Signals hin eine Initiation eines Betriebes der UV-Lichtquelle (2) zu unterbinden und/oder einen laufenden Betrieb der UV-Lichtquelle (2) zu unterbrechen.

9. Vorrichtung nach einem der vorhergehenden Ansprüche, wobei die Verbindung zwischen der Steuereinheit (3, 7) und dem mindestens einen Anwesenheitsdetektor und/oder dem mindestens einen Beschleunigungssensor (4, 8) kabelgebunden oder kabellos realisiert ist.

10. Vorrichtung nach Anspruch 9, wobei die Steuereinheit (3, 7) und der mindestens eine Anwesenheitsdetektor und/oder der mindestens eine Beschleunigungssensor (4, 8) zu einer kabellosen Datenübertragung über eine Bluetooth Datenverbindung, eine Bluetooth LE Datenverbindung, eine WLAN Datenverbindung, ZIGBEE Datenverbindung, eine LORA Datenverbindung, eine Z-Wave Datenverbindung, eine 6LowPAN Datenverbindung, eine Thread Datenverbindung, eine Sigfox Datenverbindung, eine NFC Datenverbindung, eine GSM Datenverbindung, eine UMTS Datenverbindung, eine LTE Datenverbindung und/oder eine 5G Datenverbindung ausgebildet sind.

11. Vorrichtung nach einem der vorhergehenden Ansprüche, wobei die Parameter des Betriebs der UV-Lichtquelle (2), insbesondere die Betriebsdauer, die Betriebshäufigkeit, der Startzeitpunkt sowie der Endzeitpunkt mindestens eines Betriebs, ein Betriebsintervall, eine Betriebsfrequenz, die Strahlungsintensität während mindestens eines Betriebs und/oder die Strahlungsdosis während mindestens eines Betriebes innerhalb der Vorrichtung vorprogrammiert und/oder durch einen Benutzer der Vorrichtung einstellbar und/oder veränderbar sind.

12. Vorrichtung nach einem der vorhergehenden Ansprüche, wobei die Vorrichtung ferner einen Datenspeicher umfasst und dazu ausgebildet ist, Daten zu einem Betrieb der UV-Lichtquelle (2) zu protokollieren und im Speicher zu hinterlegen.

13. Vorrichtung nach Anspruch 12, wobei die protokollierbaren Daten Informationen zu einem Beginn eines Betriebs der UV-Lichtquelle (2), zu einer Beendigung eines Betriebs der UV-Lichtquelle (2) und/oder Daten zu der Ursache für eine Unterbrechung eines Betriebs der UV-Lichtquelle (2) umfassen.

14. Aufzugsanlage mit einer Vorrichtung nach einem der Ansprüche 1 bis 13.

15. Verfahren zur Desinfektion mindestens einer Oberfläche innerhalb des Fahrkorbs (1) einer Aufzugsanlage mit mindestens einer UV-Lichtquelle (2) sowie mindestens einem Anwesenheitsdetektor und mindestens einem Beschleunigungssensor (4, 8), wobei das Verfahren die folgenden Schritte umfasst:
- Emittieren (S1) von UV-Licht auf die zu desinfizierende Oberfläche des Fahrkorbs (1) mittels der mindestens einen UV-Lichtquelle;
- Erkennen (S2) der Anwesenheit mindestens eines Lebewesens innerhalb des Fahrkorbs (1) mittels des mindestens einen Anwesenheitsdetektors;
- Erkennen (S3) der horizontalen und/oder vertikalen Bewegung der Aufzugsanlage mittels des mindestens einen Beschleunigungssensors (4, 8),
**gekennzeichnet durch** das
- Unterbrechen (S4) der Emission des UV-Lichtes, sowohl auf den Empfang des die Anwesenheit des Lebewesens indizierenden Sensorsignals, wie auch wenn mittels des mindestens einen Beschleunigungssensors (4, 8) eine horizontale und/oder vertikale Bewegung der Aufzugsanlage erkannt wird.

## Claims

1. A device for disinfecting at least one surface within the car (1) of an elevator system, the device comprising:
at least one UV light source (2) which is formed to emit UV light for disinfecting the surface within the car (1);
at least one presence detector which is formed to detect the presence of at least one living being inside the car (1) and, in the case of detection of the presence of at least one living being inside the car (1), to generate a sensor signal indicating the presence of the living being;
at least one acceleration sensor (4, 8) which is formed to detect when the device moves horizontally and/or vertically and to output a sensor signal indicating this movement; and
at least one control unit (3, 7) connected to the at least one presence detector and the at least one acceleration sensor (4, 8) and the at least one UV light source (2),
**characterized in that**,
the aforementioned device is formed to control the operation of the UV light source (2) and to prevent an initiation of an operation of the UV light source (2) in response to both the reception of the sensor signal indicating the presence of the living being and the reception of the sensor signal indicating the movement and/or to interrupt an ongoing operation of the UV light source (2).

2. The device according to Claim 1, wherein the at least one presence detector comprises at least one movement sensor (5, 10) which is formed for detecting a movement of at least one living being present inside the car (1).

3. The device according to Claim 2, wherein the at least one movement sensor (5, 10) is formed for emitting and/or receiving electromagnetic radiation, in particular radiation in the high-frequency range, in the microwave range, and/or in the ultrasonic range.

4. The device according to Claim 2 or 3, wherein the at least one movement sensor (5, 10) comprises a Doppler radar, an ultrasonic motion detector, a pyroelectric sensor, and/or a photocell motion detector.

5. The device according to any one of the preceding claims, wherein the at least one presence detector comprises at least one non-motion sensor (6, 9) which is formed to detect the presence of at least one unmoving living being inside the car (1).

6. The device according to Claim 5, wherein the at least one non-motion sensor (6, 9) comprises a laser scanner, a stereo camera a 3D camera, a time-of-flight 3D camera, and/or an active triangulation system.

7. The device according to Claim 1, wherein the at least one acceleration sensor (4, 8) is configured as an accelerometer, in particular as a 3-axis accelerometer.

8. The device according to Claim 1 or 7, wherein the control unit (3, 7) is further formed to prevent an initiation of the UV light source (2) and/or to interrupt an ongoing operation of the UV light source (2) in response to the reception of the signal indicating the movement of the elevator system.

9. The device according to any one of the preceding claims, wherein the connection between the control unit (3, 7) and the at least one presence detector and/or the at least one acceleration sensor (4, 8) is realized in a wired or wireless manner.

10. The device according to Claim 9, wherein the control unit (3, 7) and the at least one presence detector and/or the at least one acceleration sensor (4, 8) are formed for wireless data transmission via a Bluetooth data connection, a Bluetooth LE data connection, a WLAN data connection, a ZIGBEE data connection, a LORA data connection, a Z-wave data connection, a 6LowPAN data connection, a Thread data connection, a Sigfox data connection, an NFC data connection, a GSM data connection, a UMTS data connection, an LTE data connection, and/or a 5G data connection.

11. The device according to any one of the preceding claims, wherein the parameters of the operation of the UV light source (2), in particular the operation duration, the operation rate, the starting point as well as the end point of at least one operation, an operation interval, an operation frequency, the radiation intensity during at least one operation and/or the radiation dose during the at least one operation within the device are preprogrammed within the device and/or are able to be set and/or changed by a user of the device.

12. The device according to any one of the preceding claims, wherein the device further comprises a data memory and is formed to record data about an operation of the UV light source (2) and store them in the memory.

13. The device according to Claim 12, wherein the recordable data comprise information about a beginning of an operation of the UV light source (2), about an ending of the operation of the UV light source (2) and/or data about the cause of an interruption of an operation of the UV light source (2).

14. An elevator system having a device according to any one of Claims 1 to 13.

15. A method for disinfecting at least one surface within the car (1) of an elevator system having at least one UV light source (2) as well as at least one presence detector and at least one acceleration sensor (4, 8), wherein the method comprises the following steps:
- emitting (S1) UV light onto the surface of the car (1) to be disinfected by means of the at least one UV light source;
- detecting (S2) the presence of at least one living being inside the car (1) by means of the at least one presence detector;
- detecting (S3) the horizontal and/or vertical movement of the elevator system by means of the at least one acceleration sensor (4, 8),
**characterized by** the
- interrupting (S4) of the emission of the UV light, both in response to the reception of the sensor signal indicating the presence of the living being as well as when a horizontal and/or vertical movement of the elevator system is detected by means of the at least one acceleration sensor (4, 8).

## Revendications

1. Dispositif de désinfection d'au moins une surface à l'intérieur de la cabine (1) d'un système d'ascenseur, le dispositif comprenant :
au moins une source de lumière UV (2), qui est conçue pour émettre de la lumière UV pour la désinfection de la surface à l'intérieur de la cabine (1) ;
au moins un détecteur de présence, qui est conçu pour détecter la présence d'au moins un être vivant à l'intérieur de la cabine (1) et, en cas de détection de la présence d'au moins un être vivant à l'intérieur de la cabine (1), générer un signal de capteur indiquant la présence de l'être vivant ;
au moins un capteur d'accélération (4, 8), qui est conçu pour détecter quand le dispositif se déplace horizontalement et/ou verticalement et fournir en sortie un signal de capteur indiquant ce déplacement ; et
au moins une unité de commande (3, 7) reliée à l'au moins un détecteur de présence et à l'au moins un capteur d'accélération (4, 8) et à l'au moins une source de lumière UV (2),
**caractérisé en ce que**
le dispositif susmentionné est conçu pour commander le fonctionnement de la source de lumière UV (2) et, aussi bien lors de la réception du signal de capteur indiquant la présence de l'être vivant que lors de la réception du signal de capteur indiquant le déplacement, empêcher un démarrage d'un fonctionnement de la source de lumière UV (2) et/ou interrompre un fonctionnement en cours de la source de lumière UV (2).

2. Dispositif selon la revendication 1, dans lequel l'au moins un détecteur de présence comprend au moins un capteur de mouvement (5, 10) qui est conçu pour détecter un mouvement d'au moins un être vivant présent à l'intérieur de la cabine (1).

3. Dispositif selon la revendication 2, dans lequel l'au moins un capteur de mouvement (5, 10) est conçu pour émettre et/ou recevoir un rayonnement électromagnétique, en particulier un rayonnement dans le domaine des hautes fréquences, dans le domaine des micro-ondes et/ou dans le domaine ultrasonore.

4. Dispositif selon la revendication 2 ou 3, dans lequel l'au moins un capteur de mouvement (5, 10) comprend un radar Doppler, un détecteur de mouvement à ultrasons, un capteur pyroélectrique et/ou un détecteur de mouvement à cellule photoélectrique.

5. Dispositif selon l'une quelconque des revendications précédentes, dans lequel l'au moins un détecteur de présence comprend au moins un capteur de non-mouvement (6, 9) qui est conçu pour détecter la présence d'au moins un être vivant qui ne bouge pas à l'intérieur de la cabine (1).

6. Dispositif selon la revendication 5, dans lequel l'au moins un capteur de non-mouvement (6, 9) comprend un détecteur à laser, une caméra stéréoscopique, une caméra 3D, une caméra 3D temps de vol et/ou un système de triangulation actif.

7. Dispositif selon la revendication 1, dans lequel l'au moins un capteur d'accélération (4, 8) est réalisé sous la forme d'un accéléromètre, en particulier un accéléromètre à 3 axes.

8. Dispositif selon la revendication 1 ou 7, dans lequel l'unité de commande (3, 7) est en outre conçue pour, lors de la réception du signal indiquant le déplacement de l'ascenseur, empêcher un démarrage d'un fonctionnement de la source de lumière UV (2) et/ou interrompre un fonctionnement en cours de la source de lumière UV (2).

9. Dispositif selon l'une quelconque des revendications précédentes, dans lequel la liaison entre l'unité de commande (3, 7) et l'au moins un détecteur de présence et/ou l'au moins un capteur d'accélération (4, 8) est réalisée par câble ou sans câble.

10. Dispositif selon la revendication 9, dans lequel l'unité de commande (3, 7) et l'au moins un détecteur de présence et/ou l'au moins un capteur d'accélération (4, 8) sont conçus pour une transmission de données sans câble par le biais d'une liaison de données Bluetooth, d'une liaison de données Bluetooth LE, d'une liaison de données WLAN, d'une liaison de données ZIGBEE, d'une liaison de données LORA, d'une liaison de données Z-Wave, d'une liaison de données 6LowPAN, d'une liaison de données Thread, d'une liaison de donnée Sigfox, d'une liaison de données NFC, d'une liaison de données GSM, d'une liaison de données UMTS, d'une liaison de données LTE et/ou d'une liaison de données 5G.

11. Dispositif selon l'une quelconque des revendications précédentes, dans lequel les paramètres du fonctionnement de la source de lumière UV (2), en particulier la durée de fonctionnement, la cadence de fonctionnement, l'instant de démarrage ainsi que l'instant de fin d'au moins un fonctionnement, un intervalle de fonctionnement, une fréquence de fonctionnement, l'intensité de rayonnement pendant au moins un fonctionnement et/ou la dose de rayonnement pendant au moins un fonctionnement sont préprogrammés dans le dispositif et/ou peuvent être réglés et/ou modifiés par un utilisateur du dispositif.

12. Dispositif selon l'une quelconque des revendications précédentes, dans lequel le dispositif comprend en outre une mémoire de données et est conçu pour journaliser des données relatives à un fonctionnement de la source de lumière UV (2) et les enregistrer dans la mémoire.

13. Dispositif selon la revendication 12, dans lequel les données pouvant être journalisées comprennent des informations relatives au début d'un fonctionnement de la source de lumière UV (2), à la fin d'un fonctionnement de la source de lumière UV (2) et/ou des données relatives à la cause d'une interruption d'un fonctionnement de la source de lumière UV (2).

14. Système d'un ascenseur comportant un dispositif selon l'une quelconque des revendications 1 à 13.

15. Procédé de désinfection d'au moins une surface à l'intérieur de la cabine (1) d'un système d'un ascenseur avec au moins une source de lumière UV (2) ainsi qu'au moins un détecteur de présence et au moins un capteur d'accélération (4, 8), le procédé comprenant les étapes suivantes :
- émission (S 1) de lumière UV sur la surface à désinfecter de la cabine (1) au moyen de l'au moins une source de lumière UV ;
- détection (S2) de la présence d'au moins un être vivant à l'intérieur de la cabine (1) au moyen de l'au moins un détecteur de présence ;
- détection (S3) du déplacement horizontal et/ou vertical de l'ascenseur au moyen de l'au moins un capteur d'accélération (4, 8),
**caractérisé par**
- l'interruption (S4) de l'émission de la lumière UV, aussi bien lors de la réception du signal de capteur indiquant la présence de l'être vivant que lorsqu'un déplacement horizontal et/ou vertical de l'ascenseur est détecté au moyen de l'au moins un capteur d'accélération (4, 8).
